# EUROPÄISCHE PATENTANMELDUNG

(11) **EP 1 736 907 A2**
(43) Veröffentlichungstag der Anmeldung: **27.12.2006**
(21) Anmeldenummer: 06114724.5
(22) Anmeldetag: 30.05.2006
(51) Int. Cl.: G06F 19/00

(54) **Verbesserung von Messdaten-Erfassung- und Bild-Rekonstruktion bei MR-Bildern**

(30) Priorität: 10.06.2005 DE 102005026890
(71) Anmelder: SIEMENS AKTIENGESELLSCHAFT, 80333 München (DE)
(72) Erfinder: Hagen, Ulrich, 91352, Trailsdorf (DE); Heid, Oliver, 91710, Gunzenhausen (DE)

(57) **Zusammenfassung**

Die Erfindung betrifft ein Verfahren und eine Vorrichtung zur Optimierung eines kombinierten Systems für die Erfassung von magnet-resonanz-tomographischen Messdaten (MD) und eines Bild-Rekonstruktions-Prozesses. Dabei wird der Bild-Rekonstruktions-Prozess bereits während der Erfassung der Messdaten (MD) ausgeführt, indem der Berechnungs-Prozess in Berechnungs-Pakete (12-i) zerlegt wird und indem Regeln definiert werden, die festlegen, welche Voraussetzungen für die Ausführung des jeweiligen Berechnungs-Paketes (12-i) erfüllt sein müssen. Daraufhin wird der Berechnungs-Prozess in eine Ablauf-Struktur reorganisiert, basierend auf den Berechnungs-Paketen (12-i) und auf den Regeln. Daraufhin wird der Berechnungs-Prozess anhand der generierten Ablauf-Struktur synchronisiert zu dem Erfassungs-Prozess gesteuert.
Die Regeln beschreiben den Berechnungs-Prozess vollständig, d.h. inklusive aller Berechnungs-Paketen und deren logischen Abhängigkeiten. Dadurch wird die Stabilität des Berechnungsprozesses vollständig unabhängig von der zeitlichen Reihenfolge der Messdaten-Erfassung.

## Beschreibung

Die vorliegende Erfindung liegt auf dem Gebiet der Bildverarbeitung, insbesondere auf dem Gebiet der Bild-Rekonstruktion.

Das hauptsächliche Anwendungsgebiet sind Systeme für medizinische Bilddaten, die von einem Magnet-Resonanz-Tomographen (kurz: MRT - auch Kernspin-Tomograph genannt) erfasst worden sind. Die Magnet-Resonanz-Tomographie ist ein modernes Schnittbild-Verfahren, das zum einen die kernmagnetische Resonanz ausnutzt und zum anderen neben den starken Magnetfeldern eine hochfrequente Strahlung einsetzt. Es können nahezu von jedem Körperteil in beliebigem Winkel und in beliebiger Richtung Schichtaufnahmen erzeugt werden. Diese so erfassten Daten liegen in digitaler Form vor und können durch den Einsatz von Computersystemen zu aussagekräftigen Bildern verarbeitet werden. Dabei werden zu den eingestrahlten Hochfrequenz-Impulsen die resultierenden Signale in dem jeweiligen Gewebe des Patienten gemessen und für die Bilderzeugung genutzt. Die durchschnittliche Untersuchungszeit liegt in etwa zwischen 20 bis 60 Minuten. Aufgrund des physikalischen Erfassungs-Prinzips der Messdaten (z.B. die Schichtbilder, die dann zu einer 3-dimensionalen Darstellung verarbeitet werden müssen) ist es notwendig, eine Vielzahl von digitalen Daten zu verarbeiten. Dies stellt hohe Anforderungen, insbesondere Anforderungen hinsichtlich der Performance, an die Verarbeitungs-Komponenten.

Doch nicht nur im Bereich der Diagnostik werden bildgebende Verfahren eingesetzt, sondern es gibt moderne Operationsverfahren, die unter dem Einsatz von bildgebenden Verfahren sozusagen online Anwendung finden. Dabei ist es zwingend, dass eine möglichst optimale Performance sowohl des ErfassungsSystems für die Messdaten als auch des Berechnungs-Prozesses für die Messdaten sichergestellt werden kann, um die Risiken für den Patienten möglichst minimal halten zu können. Beispiele solcher MR-gesteuerter Eingriffe liegen auf dem Gebiet der interventionellen Radiologie und sind z.B. Biopsien, Interventionen bei Lebertumoren, bei knöchernen Läsionen oder bei perkutanen Wirbelkörper-Biopsien. Insbesondere bei der interventionellen Radiologie ist es unabdingbare Voraussetzung, dass eine optimierte Bildverarbeitung bzw. Bildgebung in Echtzeit sichergestellt werden kann, um die Genauigkeit des Eingriffes planen und um höchste Patienten-Sicherheit gewährleisten zu können.

Aus dem Stand der Technik sind bereits Verfahren zur Effizienzverbesserung bekannt. So offenbart der Artikel "Efficient visualization of large medical image Datasets on standard PC hardware", Proceedings of the symposium on Data visualisation, ACM International Conference Proceeding Series; Vol.40 Seiten 135 - 140 aus dem Jahre 2003 von Pekar,V. at al. einen hardware-basierten Ansatz, um medizinische Bilddaten effizienter darstellen zu können. Hier wird ein Algorithmus vorgeschlagen, der auf bereits erfasste medizinische Bilddaten angewendet wird und darauf abzielt, eine effizientere Visualisierung der Bilddaten zu ermöglichen, indem eine Speicher-Caching-Strategie modifiziert wird. Bei dem dort offenbarten Algorithmus ist es vorgesehen, den Datensatz so zu zerlegen, dass er auf die Größe des Cache angepasst wird. Dies wird auch über einen Parallelverarbeitungsansatz erreicht. Diese Druckschrift offenbart jedoch keine geeignete interdependente Verschachtelungs-Strategie für einen kombinierten Erfassungs- und Berechnungsprozess, bei dem der spätere Berechnungsprozess so zerlegt wird, dass ein optimales Ergebnis im Hinblick auf die Reihenfolge der Datenerfassung erzielt werden kann.

Darüber hinaus offenbart die US 2004/0223003 A1 ein Bildverarbeitungsverfahren und einen zugehörigen Bildgenerator, bei dem ein Gesamtbild in Bildteile zerlegt wird, die jeweils in parallel laufenden Threads und darin vorgesehenen Rendering-Maschinen und Merge-Maschinen verarbeitet werden. Mit dem in dieser Druckschrift vorgeschlagenen Ansatz kann zwar die Verarbeitungsgeschwindigkeit zur Darstellung eines Bilddatensatzes gesteigert werden, er gibt jedoch keinen Hinweis auf einen kombinierten Erfassungs- und Berechnungsprozess, wobei der Berechnungsprozess so strukturiert wird, dass er optimal auf den Erfassungsprozess, insbesondere auf die Reihenfolge der erfassten Bilddaten, ausgelegt ist.

Ein zentraler Faktor von wesentlicher Bedeutung liegt dabei in der rechner-gestützten Erfassung und Verarbeitung der Bilddaten. Unabdingbare Voraussetzung ist es deshalb, dass sichergestellt werden kann, dass es nicht zu Beeinträchtigungen oder Einschränkungen aufgrund der Bildgebung kommt. Des Weiteren ist es wichtig, die Bilddaten-Erfassung (im obigen Beispiel durch die Modalität des Magnet-Resonanz-Tomographen), insbesondere die Scan-Kontrolle, so zu steuern, dass die darauf basierende Bildrekonstruktion und Bildauswertung zeitoptimiert erfolgen können.

Das hauptsächliche Anwendungsgebiet der vorliegenden Erfindung betrifft deshalb insbesondere ein Verfahren und eine Vorrichtung zur Optimierung eines kombinierten Erfassungs- und Berechnungs-Prozesses von Bilddaten, insbesondere von MRT-Bilddaten. Insbesondere soll der kombinierte Prozess hinsichtlich der Performance, der Stabilität und/oder der Flexibilität verbessert werden.

Eine Magnet-Resonanz-Tomographie-Anlage umfasst üblicherweise einen Grundfeld-Magneten, der ein zeitkonstantes Magnetfeld erzeugt, um die Atomkerne in dem zu untersuchenden Körper bzw. Körperteil zu polarisieren. Darüber hinaus gibt es einen Sender für hochfrequente Impulse und entsprechende Empfänger, die zum Signalempfang eingesetzt werden. Ein Anlagenrechner sorgt zum Einen für eine optimale Steuerung der Messdaten-Erfassung und zum Anderen wird er für die Verwaltung der Messdaten, die Weiterverarbeitung der Daten bzw. für die Rekonstruktion von Bildern aus den erfassten Messdaten eingesetzt. Da die weitere Berechnung von Bilddaten bzw. die Rekonstruktion von Bildern auf der Erfassung der jeweiligen Messdaten basiert, ist es notwendig, dass bestimmte Bereiche von Messdaten in einer zeitlich vorgelagerten Phase erfasst werden, um später für die Rekonstruktion verarbeitet werden zu können. Es würde jedoch nachteiliger Weise zu nicht tragbaren Verzögerungen führen, wenn der Messvorgang erst vollständig ausgeführt werden müsste, bevor mit der anschließenden Rekonstruktion begonnen werden kann. Ein zentraler, für die Güte solcher Systeme entscheidender Punkt liegt in dem zeitlichen Ineinandergreifen zwischen dem Erfassungsprozess einerseits und dem anschließenden Berechnungsprozess andererseits.

Aus dem Stand der Technik ist eine kombinierte Erfassung und Berechnung von Messdaten bekannt. Diese im Stand der Technik bekannten Lösungen basieren jedoch auf einer pipeline-artigen, seriellen Verarbeitung von Eingangsdaten. Die Bilddaten werden auf eine bestimmte Weise und in einer bestimmten Reihenfolge (d.h. in einer bestimmten Mess-Sequenz) erfasst. Üblicherweise kann diese Mess-Sequenz, also die Reihenfolge der Erfassung der Messdaten, konfiguriert werden, indem die Hardware des MR-Scanners auf bestimmte Weise angesteuert wird. Nachteiligerweise ist jedoch der Berechnungsprozess nicht flexibel anpassbar. Insbesondere ist es bisher nicht möglich, die Konfiguration des Berechnungs-Prozesses zu ändern. Bisherige Systeme sind also abhängig von einer expliziten und zusätzlichen Synchronisation zwischen dem Rekonstruktions-Prozess und dem Erfassungs-Prozess und/oder von einer vorgegebenen zeitlichen Mess-Reihenfolge. Die Fehlerfreiheit und damit die Güte des Gesamt-Systems hängt damit nachteiligerweise von einer zusätzlichen Aufgabe ab, nämlich der expliziten Synchronisation und/oder der Einhaltung der notwendigen Mess-Reihenfolge. Kann diese Aufgabe nur mangelhaft ausgeführt werden, kann das Ergebnis der Bild-Rekonstruktion ebenfalls mangelhaft oder sogar unbrauchbar (in qualitativer und/oder zeitlicher Hinsicht) sein.

Darüber hinaus gibt es eine Vielzahl von Berechnungs-Prozessen, insbesondere von Rekonstruktions-Prozessen, die in diesem Zusammenhang eingesetzt werden können. Als nachteilig erweist es sich, dass es keine einheitliche und/oder keine vollständige Beschreibung dieser Berechnungs-Prozesse gibt.

Von daher war es bisher nicht möglich, die Berechnungs-Prozesse an die Erfordernisse des Erfassungs-Prozesses anzupassen. Im Gegenteil musste in bestimmten Situationen der Erfassungs-Prozess, insbesondere die Reihenfolge der Messdaten-Erfassung, an die Erfordernisse des Rekonstruktions-Prozesses angepasst werden. Dies ist aber nur sehr eingeschränkt möglich, da es insbesondere bei der MR-Bilddatenerfassung eine Vielzahl von physikalischen Voraussetzungen gibt, die eingehalten werden müssen und die somit die Konfigurationsfreiheit einschränken. Die Einflussmöglichkeiten und das Ineinandergreifen der beiden Prozesse ist bei den bekannten Systemen von daher suboptimal.

Aufgabe der vorliegenden Erfindung ist es deshalb, einen Weg aufzuzeigen, mit dem die Performance, die Stabilität und/oder die Flexibilität eines kombinierten Erfassungs- und Berechnungs-Prozesses verbessert werden kann und der es erlaubt, soviel wie möglich von der Zeit, die für die Datenerfassung notwendig ist, für die Berechnung bzw. für den Berechnungs-Prozess zu nutzen. Darüber hinaus sollen alle grundsätzlich möglichen und einsetzbaren Berechnungs-Prozesse, deren logische Abhängigkeiten und Parallelitäten einfach und standardisiert beschrieben werden können.

Diese Aufgabe wird durch die Merkmale der beiliegenden Hauptansprüche gelöst.

Die Aufgabe wird insbesondere durch ein Verfahren zur Optimierung eines kombinierten Erfassungs- und Berechnungs-Prozesses gelöst, wobei der Erfassungs-Prozess dazu bestimmt ist, Messdaten zu erfassen und wobei der Berechnungs-Prozess dazu bestimmt ist, parallel dazu auf diesen erfassten Messdaten weitere Berechnungen auszuführen, wobei der Datenaustausch zwischen dem Erfassungs-Prozess und dem Berechnungs-Prozess über eine geeignete Schnittstelle erfolgt, mit folgenden Verfahrensschritten:
- Zerlegen zumindest des Berechnungs-Prozesses in Berechnungs-Pakete;
- Spezifizieren eines Regelsatzes bzw. von Regeln für jedes Berechnungs-Paket, indem festgelegt wird, welche logischen Abhängigkeiten zwischen den Berechnungs-Paketen bestehen und insbesondere, welche Voraussetzungen für die Ausführung des jeweiligen Berechnungs-Paketes erfüllt sein müssen und/oder welche und wie viele Messdaten bereits erfasst sein müssen;
- Restrukturieren des Berechnungs-Prozesses in eine Ablaufstruktur basierend auf den Berechnungs-Paketen und auf deren Regeln;
- Steuern des Berechnungs-Prozesses anhand der Ablaufstruktur, indem automatisch zum frühestmöglichen Zeitpunkt das jeweilige Berechnungs-Paket ausgeführt wird.

In der bevorzugten Ausführungsform der Erfindung sind die Messdaten magnet-resonanz-tomographische Messdaten, die über einen Rekonstruktions-Prozess in Bilder verarbeitet werden. Der Berechnungs-Prozess ist hier also der Prozess der Rekonstruktion bzw. der Bildgenerierung. Es ist jedoch möglich, die vorliegende Erfindung ebenso auf andere Messdaten und/oder auf andere Prozesse anzuwenden, die eine Weiterverarbeitung von Messdaten umfassen, wobei die Weiterverarbeitung möglichst parallel und synchronisiert zur Erfassung der Messdaten erfolgen soll. Beispielhaft seien in diesem Zusammenhang erwähnt: die Erfassung von Fertigungsprozess-Daten und die anschließende Prozesssteuerung, die Erfassung von Versuchs- bzw. Labordaten und die anschließende Auswertung dieser Daten oder die Erfassung von Systemparametern und die anschließende Nutzung dieser Daten zum Zwecke der Konfiguration oder Diagnose.

Darüber hinaus muss der Erfassungsprozess nicht notwendigerweise für die Erfassung von Messdaten ausgelegt sein. Ebenso sind andere Akquisitionsmodalitäten der Daten denkbar, wie z.B. das Einlesen der Daten aus einem Speicher oder aus einer Datenbank. Allgemein sind unter dem Begriff Messdaten Eingangsdaten jeder Art zu verstehen.

Bekannte Systeme auf dem Gebiet der kombinierten MR-Messdaten-Erfassung und -Bildberechnung stellten bisher lediglich darauf ab, möglichst schnelle Algorithmen zur Berechnung eines Ergebnisses (also Algorithmen für den Berechnungsprozess) zu implementieren. Dieses Vorgehen hat jedoch eine natürliche Beschränkung hinsichtlich der Performance, da der Flaschenhals in der Kombination der beiden Prozesse liegt. Der Rekonstruktions-Prozess erfordert es, dass bestimmte Messdaten bereits erfasst sind und somit Grundlage für eine weitere Berechnung sein können. Als sinnvoll erweist es sich, auf einer in etwa ähnlich lang ausgelegten Erfassungs- und Berechnungszeit aufzusetzen.

Ein wesentlicher Vorteil der vorliegenden Erfindung liegt deshalb in dem Ansatz, die MR-Datenerfassung und die MR-Bildberechnung so zu parallelisieren und zu synchronisieren, dass möglichst viele Aufgaben des Rekonstruktionsvorganges bereits bei der Messdaten-Erfassung ausgeführt werden können.

Dies wird erfindungsgemäß ermöglicht, indem die beiden Prozesse - der Messdaten-Erfassungs-Prozess und der Bildberechnungs-Prozess (oder der Rekonstruktions-Prozess im obigen Beispiel) - in kleinere Sub-Prozesse zerlegt wird, in so genannte Berechnungs-Pakete. Darüber hinaus wird festgelegt, welche Voraussetzungen erfüllt sein müssen, dass das jeweilige Berechnungs-Paket ausgeführt werden kann. Dies wird üblicherweise durch parametrisierte Regeln festgelegt, in denen alle logischen Abhängigkeiten umfasst sind. Zeitliche Abhängigkeiten sind hier nicht erfasst. Dies hat den Vorteil, dass das Verfahren flexibel und unabhängig von der zeitlichen Erfassungs-Sequenz der Daten ist.

Die Regeln beschreiben den Berechnungs-Prozess vollständig, d.h. inklusive aller Berechnungs-Paketen und deren logischen Abhängigkeiten. Dadurch wird die Stabilität des Berechnungsprozesses vollständig unabhängig von der zeitlichen Reihenfolge der Messdaten-Erfassung.

Bei dem erfindungsgemäßen Vorgehen ist also die Reihenfolge der Messdaten-Erfassung beliebig. Die Verarbeitung einzelner Berechnungs-Pakete wird zeitlich verändert, wenn sich die Reihenfolge der Messdatenerfassung verändert. Insofern kann die Berechnung unabhängig von der Erfassung gesteuert werden.

Erfindungsgemäß organisiert sich das Verfahren selbst. Es kann vorteilhafterweise sichergestellt werden, dass das Verfahren so schnell wie möglich terminiert. Anhand der adaptiv generierten Ablauf-Struktur können alle Berechnungen durchgeführt werden, die zu diesem Zeitpunkt (bzw. für die bisher erfassten Daten) möglich sind. Der Berechnungsprozess ist dabei nicht von einer zeitlichen Erfassungsreihenfolge abhängig.

Folgende Beispiele können für Voraussetzungen, die für die Ausführung eines Berechnungs-Paketes erfüllt sein müssen, angeführt werden:
- Eine bestimmte Gruppe von Messdaten muss erfasst sein;
- ein anderes Berechnungs-Paket muss bereits ausgeführt worden sein;
- Ergebnisse von anderen Instanzen müssen vorliegen und/oder
- die technischen Ressourcen für die Ausführung des jeweiligen Berechnungs-Paketes müssen vorhanden sein.

Aufgrund der hier vorgeschlagenen Lösung, den kombinierten Erfassungs- und Berechnungs-Prozess in kleinere Einheiten zu zerlegen und für den Berechnungs-Prozess eine Ablauf-Struktur zu generieren, die bereits eine Synchronisation zwischen den beiden Prozessen impliziert, ist es vorteilhafter Weise möglich, moderne Rechner-Architekturen und z.B. 64-Bit-Multiprozessor-Plattformen auszunutzen, so dass ein maximaler Parallelisierungsgrad erreicht werden kann. Vorteile der modernen Rechner-Architekturen können somit effizient ausgenützt werden, indem mehrere Teilaufgaben in parallelen Threads eines Multiprozessor-Systems abgearbeitet werden können. In der bevorzugten Ausführungsform ist das Verfahren deshalb für ein Multiprozessor-System ausgelegt. Jeder Berechnungs-Prozess oder eine Gruppe von Berechnungs-Prozessen wird auf mehrere gleichläufige Threads aufgeteilt. Damit kann eine moderne Rechner-Architektur effizient ausgenützt werden, indem die generierte Ablauf-Struktur an die Anzahl der verfügbaren CPU's angepasst werden kann. Alternative Lösungen basieren auf einer Single-Prozessor Maschine. Die grundlegende Idee der vorliegenden Erfindung kann jedoch auch hier angewendet werden. Insbesondere ist es auch bei einer Single-Prozessor Maschine möglich, möglichst viele Berechnungsaufgaben in die Messung hinein vorzuziehen. Damit kann das Verfahren flexibel an die jeweilige Rechnerarchitektur angepasst werden.

Auf dem Gebiet der MR-Rekonstruktion existieren eine Vielzahl von möglichen und einsetzbaren Algorithmen. Die vorliegende Lösung ist auf keinen bestimmten Algorithmus beschränkt und kann auf beliebige Berechnungs-Prozesse angewendet werden, indem eine Datenstruktur bereitgestellt wird, in die der Berechnungs-Prozess jeweils restrukturiert wird. Damit kann eine spezifische und optimal ausgelegte Notation für die Beschreibung der Bildberechnung während der Bilderfassung bereitgestellt werden.

Üblicherweise ist das erfindungsgemäße Verfahren in zwei zeitliche Abschnitte unterteilt:
1. Eine Vorbereitungsphase, in der der Berechnungs-Prozess in Berechnungs-Pakete zerlegt wird, in der für jedes Berechnungs-Paket spezifiziert wird, welche Voraussetzungen für seine Ausführung erfüllt sein müssen bzw. welche logischen Abhängigkeiten das jeweilige Berechnungs-Paket hat und in der der Berechnungs-Prozess in eine neue Struktur transformiert wird, nämlich in eine Ablauf-Struktur, die auf den definierten Berechnungs-Paketen und auf den definierten Abhängigkeiten und/oder Voraussetzungen basiert.
2. Eine Ausführungsphase: Diese Ausführungsphase dient zur Ausführung der Erfassung und der Berechnung anhand der generierten Ablauf-Struktur und zum Austausch der jeweiligen Daten. Während des Erfassungsvorganges, insbesondere während des Scannens des MRT-Gerätes, werden parallel möglichst viele Berechnungs-Pakete ausgeführt.

In der Vorbereitungsphase können bestimmte Ereignisse definiert werden, die eine Darstellung des Zwischenergebnisses veranlassen sollen. Beispielsweise wenn ein MRT-Verfahren von mehreren Körperteilen eines Patienten ausgeführt werden soll, kann eingestellt werden, dass ein Zwischenergebnis jeweils nach Abschluss der Erfassung der Messdaten für jeweils ein Körperteil und/oder nach Rekonstruktion des Bildes für ein Körperteil angezeigt werden soll.

Üblicherweise ist die Vorbereitungsphase in zeitlicher Hinsicht der Ausführungsphase vorgelagert. Dies ist jedoch nicht zwingend, denn es ist auch möglich, die beiden Phasen zu verschmelzen. Insbesondere können Regeln auch zu einem späteren Zeitpunkt nachgeliefert werden. Dies erhöht die Flexibilität der erfindungsgemäßen Lösung deutlich.

Die Vorbereitungsphase kann einen fixen und einen dynamischen Teil umfassen. In dem dynamischen Teil können (nachgelieferte) Regeln definiert werden. Hier ist es möglich, weitere Einstellungen zu konfigurieren.

Des Weiteren ist es möglich, Prioritäten zu definieren. Alle Berechnungspakete, die z.B. ein bestimmtes Ergebnis bzw. Ergebnisobjekt erzeugen, können als höchste Priorität festgelegt werden, während andere Pakete eine untergeordnete Priorität haben. Hier werden die Berechnungspakete wahlweise zusätzlich in Hinblick auf deren Priorität gruppiert und verarbeitet.

Durch das zeitliche Vorziehen der Vorbereitungsphase ist es möglich, die CPU von zusätzlichen Aufgaben zu befreien, indem alle Definitions- und Spezifikations-Aufgaben bereits im Vorfeld erledigt werden können. In der bevorzugten Ausführungsform wird der parametrisierte Regelsatz für den Berechnungs-Prozess im Rahmen des Entwicklungsprozesses für den Berechnungsvorgang erzeugt. Üblicherweise wird der Entwickler des Rekonstruktionsprozesses den Regelsatz in Hinblick auf die Messdatenerfassung definieren. Jedoch ist das Verfahren - im Gegensatz zum Stand der Technik - robust im Hinblick auf eine suboptimal ausgelegte Erfassung der Messdaten.

Mit anderen Worten wird erfindungsgemäß der in einzelne Berechnungs-Pakete zerlegte Berechnungs-Prozess in eine Ablauf-Struktur transformiert. In dieser Ablauf-Struktur sind implizit die Vorgaben für eine Synchronisation mit dem Erfassungs-Prozess geregelt. Üblicherweise werden für jedes Berechnungs-Paket zwei Synchronisations-Punkte definiert. In einem Synchronisationspunkt sind alle logischen Abhängigkeiten des jeweiligen Paketes definiert, also insbesondere von welchen anderen Ereignissen oder Pakten das jeweilige Paket abhängig ist und welche anderen Ereignisse oder Pakete von dem jeweiligen Paket abhängen.

Ein Berechnungs-Paket kann grundsätzlich nur dann ausgeführt werden, wenn alle logischen Voraussetzungen erfüllt sind, d.h., wenn bestimmte Ereignisse eingetreten sind. Die erfolgreiche Ausführung eines Berechnungs-Prozesses kann dabei ein solches Ereignis für andere Berechnungs-Prozesse sein. Andere Beispiele für vorstehend genannte Ereignisse sind z.B. die Erfassung von Messdaten, die Vorverarbeitung der erfassten Messdaten oder die Verarbeitung von "benachbarten" Messdaten. Die Voraussetzungen, die für die Ausführung des jeweiligen Berechnungs-Paketes erfüllt sein müssen, können aus einem solchen Ereignis bestehen - sie können jedoch auch aus einer beliebigen Kombination solcher Ereignisse bestehen.

Erfindungsgemäß ist ein Mechanismus vorgesehen, der ein Berechnungs-Paket so lange von seiner Ausführung blockiert, bis alle Voraussetzungen erfüllt sind und der die Ausführung eines Berechnungs-Paket erfasst und der die Ausführung anderer Berechnungs-Pakete veranlassen kann bzw. der andere Berechnungs-Pakete von der Blockierung ausnehmen kann. Dieser Mechanismus ist in der bevorzugten Ausführungsform durch die Synchronisations-Punkte ausgebildet.

In dieser Ausführungsform gibt es grundsätzlich zwei Kategorien von Synchronisations-Punkten:
1. Blocker: Er überwacht, ob alle notwendigen Ereignisse stattgefunden haben, die als Voraussetzung für das jeweilige Berechnungs-Paket definiert worden sind. Solange nicht alle Voraussetzungen erfüllt sind, blockiert er das Paket von seiner Abarbeitung.
2. Kollektor: Dieser Synchronisations-Punkt nimmt die jeweilige Ausführung des Berechnungs-Paketes auf und/oder registriert diese aus der Sicht des jeweiligen Berechnungs-Paketes.

Typischerweise ist ein bestimmter Synchronisationspunkt zugleich Kollektor für bestimmte Berechnungspakete und Blocker für bestimmte andere Berechnungspakete.

Es gibt drei ausgezeichnete Synchronisationspunkte, nämlich den Datenerfassungs-Punkt, der die direkte Abhängigkeit von Messdaten repräsentiert, einen von vornherein unblockierten Blocker, der es erlaubt Arbeitspakete zu definieren, die von nichts abhängen, und genau einen Kollektor pro gewünschtem Berechnungsergebnis, der angibt, ob das Ergebnis korrekt berechnet wurde.

Die Interdependenzen zwischen den einzelnen Berechnungs-paketen werden in der bevorzugten Ausführungsform durch parametrisierte Regeln spezifiziert. In den Regeln sind die einzelnen Berechnungs-Pakete und deren logische Abhängigkeiten definiert. Jede Regel kann mehrfach angewendet werden; dabei ist die Anzahl der Wiederholungen jeweils Bestandteil der Regel. Die mehrfachen Wiederholungen müssen aufgelöst werden. Dies wird über einen Wiederholungs-Index erreicht.

In der bevorzugten Ausführungsform der Erfindung ist es möglich, über eine entsprechende Schnittstelle externe Programme anzuschließen, die beispielsweise für die Darstellung und Anzeige der Ablauf-Struktur ausgelegt sind. Eine Eingabedatei, die die parametrisierten Regeln umfasst, wird dann in eine graphische Darstellung, z.B. in einem gerichteten Graphen oder in einer Tabelle, abgebildet.
In der generierten Datenstruktur sind die Abhängigkeiten zwischen den einzelnen Berechnungs-Paketen zusammen mit den Synchronisations-Punkten dargestellt. Der Graph kann aus mehreren Wurzelknoten bestehen und hat grundsätzlich nur ein Blatt, nämlich das Ergebnis. Die Kanten sind die abzuarbeitenden Prozesse (die Berechnungsaufgaben) und die Knoten sind die Synchronisationspunkte.

Diese graphische Darstellung kann ausgegeben werden, um die Restrukturierung des Berechnungs-Prozesses in die Ablauf-Struktur auf Korrektheit hin zu überprüfen. Hat der Graph z.B. mehrere Blätter, so ist der Prozess inkorrekt restrukturiert worden, da nur jeweils ein Endergebnis existieren kann.

Eine Aufgabenlösung liegt deshalb in einer Datenstruktur gemäß dem beiliegenden Hauptanspruch. Diese Notation ist vollständig und umfasst alle Berechnungs-Pakete und alle Abhängigkeiten.

Sie liegt insbesondere ferner in einer Datenstruktur für einen Berechnungs-Prozess zur Verwendung bei einem Verfahren gemäß den vorstehend erwähnten Merkmalen, mit:
- einer Vielzahl von Berechnungs-Paketen, in die der Berechnungs-Prozess zerlegt ist;
- zwei Synchronisations-Punkten für jedes einzelne Berechnungs-Paket, über die jeweils definiert wird, welche Voraussetzungen bzw. welche logischen Abhängigkeiten das jeweilige Berechnungs-Paket hat;
- Zuordnung zwischen jeweils einem Berechnungs-Paket und den beiden Synchronisations-Punkten.

Eine Zuordnungsrelation wird nur dann erstellt, falls eine Zuordnung existiert. Bei einer initialen Messdaten-Erfassung und bei dem Abschluss der letzten Tätigkeit des Berechnungs-Prozesses, insbesondere nach dem Speichern des rekonstruierten Bildes, ist eine solche Zuordnung nur teilweise bzw. nicht mehr notwendig. Mit dieser Datenstruktur wird es möglich, sofort, automatisch und für jedes Berechnungspaket separat zu erfassen, welche Voraussetzungen für dessen Verarbeitung erfüllt sein müssen.

Mit der erfindungsgemäßen Datenstruktur ist es möglich, unterschiedlichste Rekonstruktions-Algorithmen in unterschiedlicher Komplexität auf standardisierte Weise einheitlich zu beschreiben und diese Beschreibung für die Parallelisierung und Synchronisation mit dem Erfassungs-Prozess zu nutzen.

Ein besonderer Vorteil der erfindungsgemäßen Lösung ist darin zu sehen, dass das Verfahren dynamisch konfigurierbar ist und adaptiv an die jeweiligen Kontext-Bedingungen angepasst werden kann, indem mögliche Änderungen in dem Arbeitsablauf bezüglich der Erfassung und/oder der Berechnung der Messdaten erfasst werden können. Insbesondere ist es möglich, die Mess-Sequenz, also die Reihenfolge der Erfassung der Messdaten abzuändern. Üblicherweise erfolgt dies über eine entsprechende Benutzer-Oberfläche. Darüber hinaus ist es möglich, einen anderen Algorithmus als Grundlage für den Berechnungs-Prozess auszuwählen und/oder andere Parameter und Einstellungen des kombinierten Erfassungs- und Berechnungs-Prozesses zu verändern. Somit ist die erfindungsgemäße Lösung grundsätzlich unabhängig von der aktuellen Organisation der Berechnungs-Pakete, von dem Erfassungsvorgang und/oder von dem aktuell ausgewählten Arbeitsablauf.

Erfindungsgemäß ist es möglich, den Berechnungs-Prozess unabhängig von der Messdaten-Erfassung (der Reihenfolge, Geschwindigkeit und/oder weiteren Parametern) zu steuern. Die Ablauf-Struktur sieht eine Vielzahl von parallel laufenden Threads vor, die jeweils einzelne Berechnungs-Pakete bearbeiten. Dabei ist die Ablauf-Struktur so ausgelegt, dass automatisch das Berechnungs-Paket ausgeführt wird, dessen Voraussetzungen erfüllt sind, insbesondere dessen Messdaten erfasst sind. Damit wird vorteilhafter Weise sichergestellt, dass die zeitliche Ausführung der einzelnen Teilaufgaben des Berechnungs-Prozesses automatisch optimiert wird, so dass ein maximaler Anteil an Berechnungsarbeit bereits während der Erfassung der Messdaten erfolgen kann.

Die Restrukturierung des Berechnungs-Prozesses umfasst eine Synchronisation zwischen Messdaten-Erfassung und Messdaten-Berechnung bzw. -Konstruktion, die sich lediglich auf die Identifikation des Datenpaketes beschränkt. Damit entsteht der wesentliche Vorteil, dass das Verfahren keiner weiteren Module für eine externe zeitliche Synchronisation bedarf und somit stabiler und weniger fehleranfällig ist. Darüber hinaus ist das Verfahren selbst-organisierend. Auch dies trägt zur deutlichen Reduzierung der Fehleranfälligkeit bei, da das Verfahren automatisch ablaufen kann und keine Fehler, etwa durch fehlerhafte Benutzerinteraktionen, entstehen können.

Bevorzugter Weise umfasst die erfindungsgemäße Lösung einen Pufferspeicher, in dem Rohdaten von der Messdaten-Erfassung abgelegt werden. Darüber hinaus ist es möglich, Zwischenergebnisse des Rekonstruktions-Prozesses abzulegen. Sobald ein Satz von Messdaten erfasst worden ist, wird er in den Pufferspeicher zwischengespeichert. Aus diesem Zwischenspeicher werden die Messdaten dann ausgelesen, wenn sie von dem jeweiligen Berechnungs-Paket angefordert werden. Darüber hinaus ist ein Speicherüberwachungs-Mechanismus vorgesehen, der dafür sorgt, dass der Erfassungs-Prozess keine weiteren Rohdaten in den Speicher pumpt, falls dieser voll ist, weil der Berechnungs-Prozess beschäftigt ist und zur Zeit keine weiteren Messdaten auslesen und weiterverarbeiten kann.

In einer komplexeren Erweiterung der Erfindung ist es möglich, den Erfassungs-Prozess nicht mehr konstant beizubehalten, sondern die Reihenfolge der Messdaten-Erfassung zu verändern. Vorzugsweise wird der Erfassungs-Prozess so gesteuert, dass die Messdaten zuerst erfasst werden, die in dem Rekonstruktions-Prozess zuerst verarbeitet werden müssen. In diesem Fall wird die Ablauf-Struktur zusammen mit den Synchronisations-Punkten ausgewertet, so dass die Berechnungs-Pakete jeweils bei Bedarf ihre notwendigen Messdaten anfordern und einen entsprechenden Messvorgang auslösen. Dies hat den Vorteil, dass weniger Rohdaten im Pufferspeicher zwischengespeichert werden müssen. Darüber hinaus trägt dies zu einer weiteren Performance-Verbesserung bei.

In der Regel erfolgt die Steuerung des Berechnungs-Prozesses anhand der erfindungsgemäß generierten Ablauf-Struktur automatisch. Dies umfasst auch das automatische Auslesen von angeforderten Messdatensätzen aus dem Pufferspeicher. Es ist also sichergestellt, dass automatisch all diejenigen Berechnungs-Pakete (also Berechnungs-Subaufgaben) ausgeführt werden, die zu dem jeweiligen Zeitpunkt (insbesondere in Bezug auf die Messdaten-Erfassung) ausführbar sind. In vorteilhaften Weiterbildungen der Erfindung werden alle oder ausgewählte Verfahrensschritte des vorstehend beschriebenen Verfahrens automatisch ausgeführt.

Eine weitere Aufgabenlösung liegt in einer Vorrichtung gemäß dem beiliegenden Hauptanspruch. Die vorstehend erwähnten Vorteile, Merkmale, alternativen Ausführungsformen, die im Zusammenhang mit den erfindungsgemäßen Verfahren erwähnt worden sind, sind entsprechend auf die erfindungsgemäße Vorrichtung anzuwenden.

Die vorstehend beschriebenen, erfindungsgemäßen Ausführungsformen des Verfahrens können auch als Computerprogrammprodukt ausgebildet sein, wobei der Computer zur Durchführung des oben beschriebenen, erfindungsgemäßen Verfahrens veranlasst wird und dessen Programmcode durch einen Prozessor ausgeführt wird.

Eine alternative Aufgabenlösung sieht ein Speichermedium vor, das zur Speicherung des vorstehend beschriebenen, computerimplementierten Verfahrens bestimmt ist und von einem Computer lesbar ist.

Darüber hinaus ist es möglich, dass einzelne Komponenten des vorstehend beschriebenen Verfahrens in einer verkaufsfähigen Einheit und die restlichen Komponenten in einer anderen verkaufsfähigen Einheit - sozusagen als verteiltes System - ausgeführt werden können.

Eine erfindungsgemäße weitere Lösung der Aufgabe liegt deshalb in einem Produkt zur Optimierung einer Erfassungs- und Berechnungseinheit, insbesondere einer Bilddatenerfassungs- und Berechnungseinheit, wobei die Erfassungseinheit Messdaten erfasst und wobei die Berechnungseinheit auf den erfassten Messdaten basiert, während parallel zur Ausführung der Berechnungseinheit weitere Messdaten von der Erfassungseinheit erfasst werden können, wobei eine Schnittstelle vorgesehen ist, über die die beiden Einheiten kommunizieren, umfassend:
- eine Vielzahl von Berechnungsmodulen, die dazu bestimmt sind, die in Berechungspakete zerlegte Aufgabe der Berechnungseinheit auszuführen und die auf verschiedene Threads aufgeteilt werden können
- zumindest eine Synchronisationseinheit, die anhand von Regeln dazu bestimmt ist, für jedes Berechnungspaket festzulegen, welche logischen Abhängigkeiten zu anderen Paketen bestehen
- einer Datenstruktur für den Ablauf der Berechnungspakete der Berechnungseinheit, basierend auf der Synchronisationseinheit
- zumindest eine Steuerungseinheit, die dazu bestimmt ist, die Ausführung der Berechnungseinheit anhand der generierten Datenstruktur zu steuern, indem automatisch zum frühestens möglichen Zeitpunkt das jeweilige Berechnungspaket ausgeführt wird, sobald dessen Synchronisationseinheit dies veranlasst, wobei das Produkt Mittel umfasst, die zur Durchführung derjenigen Einheiten der Vorrichtung nach zumindest einem der vorstehend beschriebenen Aspekte (die Aspekte können auch im Zusammenhang mit dem erfindungsgemäßen Verfahren erwähnt worden sein) eingerichtet sind, die von dem Produkt bewirkt werden, wobei zumindest ein weiteres Produkt zur Implementierung der restlichen Einheiten der Vorrichtung eingerichtet ist, so dass durch Zusammenwirken der zwei Produkte alle Aufgaben der Vorrichtung durchgeführt werden.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Figur 1: eine übersichtsartige Darstellung über einen Messdaten-Erfassungs- und Bild-Rekonstruktions-Prozess gemäß einer bevorzugten Ausführungsform der Erfindung,
- Figur 2: einen Datenfluss gemäß einer bevorzugten Ausführungsform der Erfindung,
- Figur 3: eine beispielhafte Anwendung des erfindungsgemäßen Verfahrens auf das Auslesen einer Fourier-transformierten Darstellung,
- Figur 4: eine beispielhafte Anwendung des erfindungsgemäßen Verfahrens auf die Ausführung einer phasen-encodierten Fourier-Transformation,
- Figur 5: eine beispielhafte Anwendung des erfindungsgemäßen Verfahrens auf eine Kombination der vorhergehenden Berechnungen,
- Figur 6: eine beispielhafte Anwendung des erfindungsgemäßen Verfahrens auf das Speichern eines berechneten Bildes,
- Figur 7: eine beispielhafte Darstellung einer erfindungsgemäßen Datenstruktur in Form eines Abhängigkeits-Baumes,
- Figur 8: eine übersichtsartige Darstellung über einen Ablauf gemäß einer vorteilhaften Ausführungsform der Erfindung und

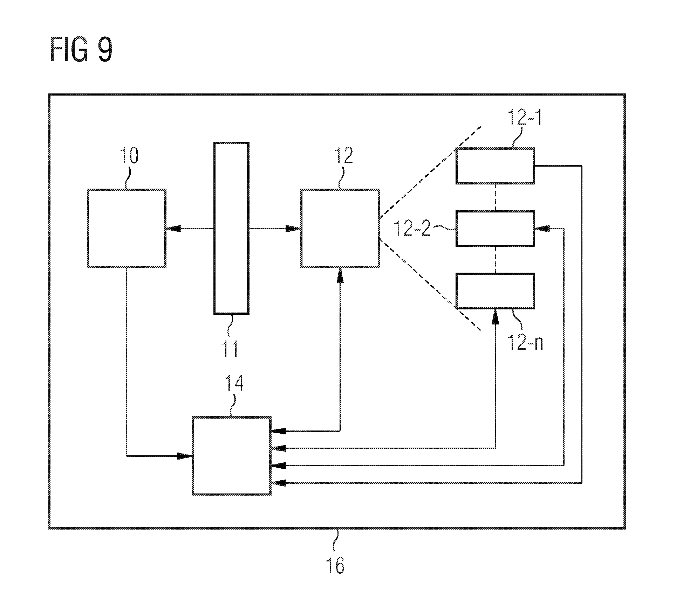

- Figur 9: eine übersichtsartige Darstellung über die wesentlichen Einheiten gemäß einer bevorzugten Ausführungsform der Erfindung.

Das hauptsächliche Anwendungsgebiet der vorliegenden Erfindung betrifft die Messdaten-Erfassung von einem Kernspin-Gerät, also so genannte MRT-Daten und den Bild-Rekonstruktions-Prozess. Grundsätzlich ist der Messdaten-Erfassungs-Prozess jedoch nicht auf Daten eines Kernspin-Gerätes begrenzt, sondern kann ebenso Daten von anderen Modalitäten, z.B. eines Computer-Tomographen, PET-Daten etc., umfassen. Ebenso ist der Berechnungs-Prozess nicht auf einen Bild-Rekonstruktions-Prozess begrenzt, sondern kann in allen Arten von Nachbearbeitung von erfassten Messdaten MD bestehen.

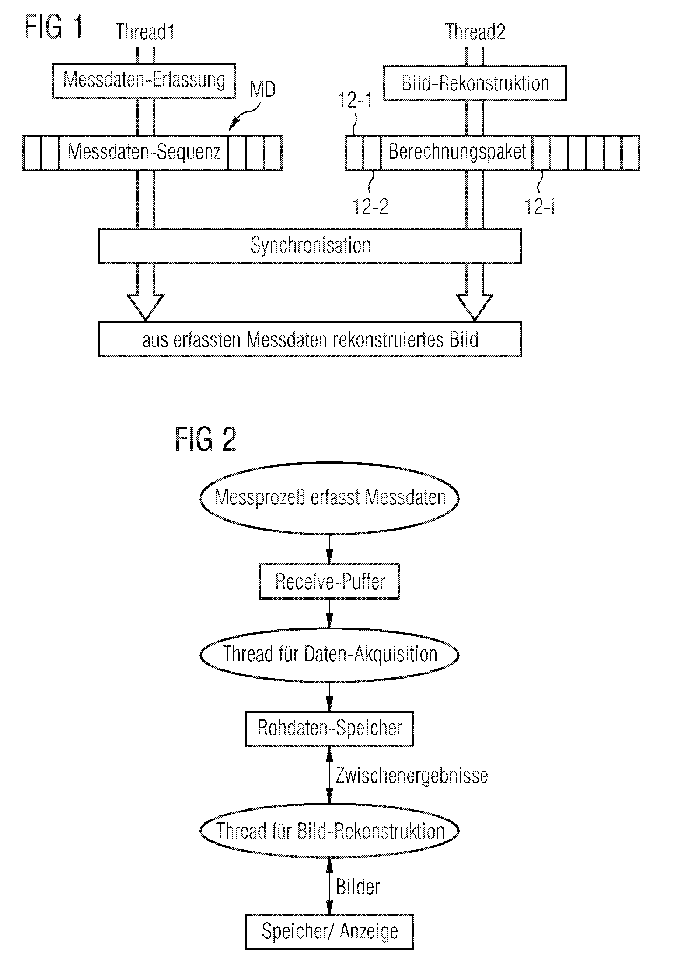

Wie in Fig.1 übersichtsartig dargestellt, handelt es sich in der bevorzugten Ausführungsform um einen kombinierten Messdaten-Erfassungs- und Bild-Rekonstruktions-Prozess. Dabei sind in der Regel zwei Threads vorgesehen, die parallel ablaufen.

Der Bild-Rekonstruktions-Prozess wird erfindungsgemäß in mehrere Berechnungs-Pakete 12-1, 12-2, ...., 12-n, zerlegt. Abhängig davon, welche Messdaten zu diesem Zeitpunkt bereits erfasst sind, wird das Berechnungs-Paket 12-i ausgeführt, dessen Voraussetzungen erfüllt sind. Insbesondere müssen alle Messdaten MD erfasst sein, die von dem jeweiligen Berechnungs-Paket 12-i verarbeitet werden müssen. Dies wird über eine erfindungsgemäße Synchronisations-Einheit 14 gewährleistet. Die Synchronisation und Parallelisierung zwischen den beiden Prozesse und gegebenenfalls noch weitere Steuerungsaufgaben übernimmt eine Steuereinheit 16. Die jeweiligen Einheiten kommunizieren über entsprechende Schnittstellen und insbesondere die Erfassungseinheit 10 und die Berechnungseinheit 12 interagieren über die Schnittstelle 11.

Im allgemeinen wird mit dem hier vorgestellten Verfahren sichergestellt, dass alle Berechnungsaufgaben ausgeführt werden können, die zu diesem Zeitpunkt ausführbar sind, d.h. insbesondere deren Messdaten bis zu diesem Zeitpunkt erfasst worden sind. Damit kann sichergestellt werden, dass der kombinierte Messdaten-Erfassungs- und Bild-Rekonstruktions-Vorgang so schnell wie möglich ein rekonstruiertes Bild erzeugen kann.

Wie in Fig.2 dargestellt, werden die Messdaten MD in der Regel von einem Kernspin-Gerät in einem Messprozess erfasst und in einen Receive-Puffer geschrieben. Der Thread für die Daten-Akquisition liest diese Daten aus dem Receive-Puffer aus und schreibt die so erfassten Daten in einen Rohdaten-Speicher. Der Thread für die Bild-Rekonstruktion liest die Daten aus dem Rohdaten-Speicher und verarbeitet diese zu Teilergebnissen, die wiederum in den Rohdaten-Speicher zwischengespeichert werden können. Sobald alle Messdaten MD erfasst worden sind und vorliegen und sobald daraufhin alle Berechnungs- bzw. Rekonstruktions-Prozesse ausgeführt worden sind, kann das Bild, bzw. können die Bilder in einem Speicher gespeichert werden oder auf einem Anzeigegerät dargestellt werden.

Eine erfindungsgemäße Datenfluss-Architektur ermöglicht es, dass der Berechnungs-Prozess, insbesondere die Bild-Rekonstruktion, bereits während der Messdaten-Erfassung erfolgt.

Vorteilhafter Weise ist das erfindungsgemäße Verfahren zur Optimierung der Performance, der Stabilität und der Flexibilität eines kombinierten Erfassungs- und Bild-Rekonstruktions-Prozesses unabhängig von der Wahl des jeweiligen Bild-Rekonstruktions-Prozesses. Es können hier also beliebige Rekonstruktions-Algorithmen verwendet werden.

### Nachfolgend wird der grundlegende Ablauf erläutert:

Der Rekonstruktions-Prozess wird in einzelne Berechnungs-Pakete 12-i zerlegt. Daraufhin wird für jedes Berechnungs-Paket 12-i definiert, welche Voraussetzungen für die Ausführung dieses Berechnungs-Paketes 12-i erfüllt sein müssen, insbesondere welche Messdaten MD erfasst sein müssen. Daraufhin wird der Berechnungs-Prozess in eine neue Datenstruktur überführt. Diese Ablauf-Datenstruktur basiert auf den Berechnungs-Paketen 12-i und deren Regeln und umfasst zwei Synchronisations-Punkte für jedes Berechnungs-Paket 12-i. Diese Verfahrensschritte können in einer Vorbereitungsphase vor der Ausführung des eigentlichen Mess- und Rekonstruktions-Vorganges ausgeführt werden.

Daran schließt sich das parallelisierte und synchronisierte Steuern des Berechnungs-Prozesses an. Der Berechnungs-Prozess wird anhand der generierten Ablauf-Struktur unabhängig von der Reihenfolge der Messdaten-Erfassung gesteuert, indem automatisch zum frühestmöglichen Zeitpunkt das jeweilige Berechnungs-Paket ausgeführt wird, dessen Synchronisations-Punkt erreicht ist. Das heißt, dass automatisch dasjenige - oder falls mehrere: diejenigen - Berechnungs-Paket(e) 12-i ausgeführt wird/werden, dessen Messdaten MD vorliegen.

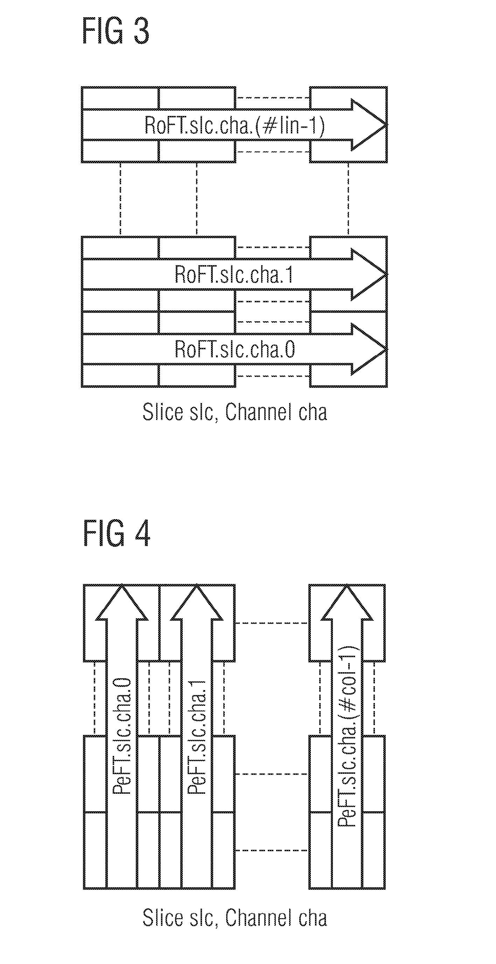

Die erfindungsgemäße Parallelisierung und Synchronisation des MR-Rekonstruktions-Prozesses soll nachstehend im Zusammenhang mit den Fig. 3 bis 6 an einem Beispiel dargestellt werden. Dieses Beispiel betrifft die Erzeugung von zweidimensionalen Bildern, die über mehrere Empfangskanäle empfangen worden sind. Die Anzahl der Schichtaufnahmen (Slices) wird mit "#slc" abgekürzt. Entsprechend werden in diesem Beispiel Linien (lines), Spalten (columns) und Kanäle (channels) mit "lin" bzw. "col" und "cha" abgekürzt.

Die Daten werden Linie für Linie über alle Kanäle gleichzeitig erfasst in unterschiedlicher Reihenfolge, was die Linien und was die Slices betrifft.

Wie in Fig.3 dargestellt, wird jede Linie einer Fourier-Transformation zugeführt. Dieser Vorgang ist als "Read-out-Fourier-Transform" gekennzeichnet (Auslesen einer Fouriertransformierten Darstellung) und ist abgekürzt mit "RoFT". Für jede Linie jedes Kanals jeder Schicht ist somit eine RoFT-Operation notwendig.

Auch die Spalten werden einer Fourier-Transformation zugeführt. Dieser Vorgang ist in Fig.4 mit "Phase-encode-Fourier-Transform" gekennzeichnet und wird abgekürzt mit "PeFT". Für jede Spalte jedes Kanals jeder Schicht ist eine PeFT-Operation notwendig.

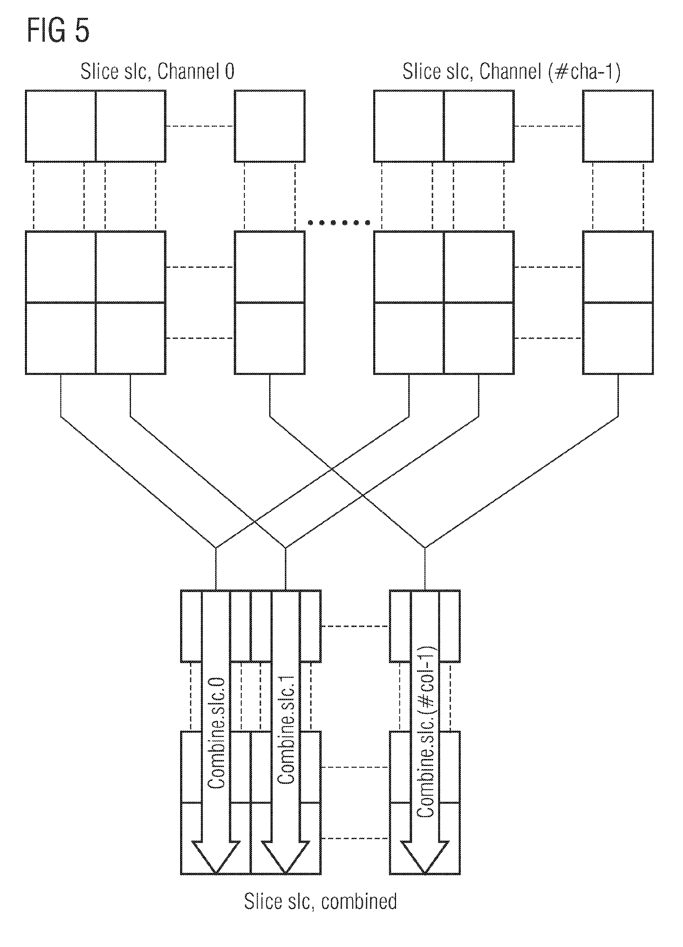

Die Bilder der Kanäle werden kombiniert, um ein Endbild für eine Schicht zu erhalten. Dieser Vorgang ist mit "Combine" gekennzeichnet. Es wird eine Spalte jeweils mit einer anderen Spalte kombiniert. Für jede Spalte jeder Schicht ist jeweils ein "Combine"-Vorgang notwendig. Der Vorgang des Kombinierens bzw. des Zusammenführens ist in Fig.5 beispielhaft dargestellt.

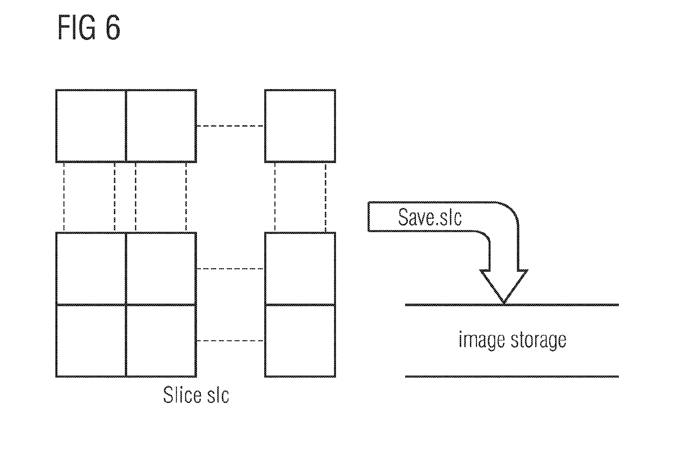

Die Schichten werden daran anschließend gespeichert. Dieser Vorgang ist beispielhaft in Fig.6 als "Save" (für Speichern) dargestellt. Für jede Schicht ist eine Speicher-Operation notwendig.

In dem eben dargestellten Beispiel handelte es sich bei dem Berechnungs-Prozess um die Verarbeitung von Fouriertransformierten Zeilen und Spalten für die Darstellung eines zweidimensionalen Bildes. Der eigentliche Algorithmus bzw. der eigentliche Berechnungs-Prozess ist in kleine Berechnungs-Pakete 12-i zerlegt worden. In obigem Beispiel kann ein Berechnungs-Paket 12-i das Auslesen einer Fouriertransformierten Darstellung einer Zeile sein (RoFT), die phasen-encodierte Fourier-Transformation (PeFT), die Kombinationen und/oder die Speichervorgänge betreffen.

Diese Berechnungs-Pakete 12-i können auf mehrere Prozessoren einer CPU aufgeteilt werden. Die Reihenfolge der Ausführung der einzelnen Bearbeitungs-Pakete 12-i wird erfindungsgemäß mit der Reihenfolge der Messdaten-Erfassung automatisch synchronisiert.

Es ist notwendig, Regeln zu definieren, um den zugrundeliegenden Berechnungs-Prozess detaillierter zu beschreiben. Die Regeln sind in der bevorzugten Ausführungsform parametrisiert und können unter Bezugnahme auf obiges Beispiel folgendermaßen dargestellt werden:
- Der RoFT-Vorgang einer Zeile kann erst dann ausgeführt werden, wenn die Messdaten MD gescannt sind;
- der PeFT-Vorgang einer Spalte kann erst dann ausgeführt werden, nachdem alle RoFT-Operationen aller Zeilen desselben Kanals und derselben Schicht fertiggestellt worden sind;
- die Kombination einer bestimmten Spalte kann erst dann ausgeführt werden, nachdem alle PeFT-Operationen dieser Spalte für alle Kanäle dieser Schicht fertiggestellt worden sind und
- die Schichtaufnahme kann erst dann gespeichert werden, nachdem alle Spalten kombiniert worden sind.

Die Regeln können in Form folgender Tabelle dargestellt werden:

| Arbeit | Blocker | Kollektor | Wiederholungen | Aufgelöste Platzhalter |
|---|---|---|---|---|
| Save.slc | Combined.slc | Saved.slc | #slc | Slc(i) |
| Combine.slc.col | PeFTed.slc.col | Combined.slc | #slc * #col | Slc (i), col(i) |
| PeFT.slc.col.cha | RoFTed.slc.cha | PeFTed.slc.col | #slc * #col * #cha | Slc (i), col(i), #cha(i) |
| RoFT.slc.lin.cha | Data Acquisition | RoFTed.slc.cha | #slc * #lin * #cha | Slc (i), lin(i), cha(i) |

Über diese parametrisierten Regeln ist es möglich, zu definieren, welche Ereignisse erfüllt sein müssen, dass bestimmte Berechnungs-Pakete 12-i ausgeführt werden können. Die erfolgreiche Ausführung eines Berechnungs-Paketes 12-i ist z.B. ein Ereignis für jeweils andere Berechnungs-Pakete 12-i. Darüber hinaus ist die Erfassung von Messdaten MD, die in dem Berechnungs-Paket 12-i verarbeitet werden sollen, ein Beispiel für ein solches Ereignis.

Dafür wird erfindungsgemäß ein Mechanismus zur Verfügung gestellt, der es ermöglicht, dass ein bestimmtes Berechnungs-Paket 12-i so lange blockiert wird und somit von der Ausführung ausgeschlossen bleibt, solange nicht alle Voraussetzungen erfüllt sind. Darüber hinaus ist es notwendig, die Ausführung eines Berechnungs-Paketes 12-i zu erfassen und die Blockierung von Berechnungs-Paketen 12-i aufzuheben. Dieser Mechanismus wird durch die Synchronisations-Punkte ausgebildet.

Für jedes Berechnungs-Paket 12-i werden in der bevorzugten Ausführungsform zwei Synchronisations-Punkte definiert.

Es gibt grundsätzlich zwei Arten von Synchronisations-Punkten:
- So genannte Blocker und
- so genannte Kollektoren.

Ein Synchronisations-Punkt gilt als Blocker, wenn er dazu dient, ein Berechnungs-Paket 12-i von dessen Ausführung zu blockieren (bzw. auszuschließen), falls noch nicht alle Voraussetzungen erfüllt sind. Ein Synchronisations-Punkt gilt als Kollektor, falls er die Ausführung des jeweiligen Berechnungs-Paketes 12-i erfasst bzw. registriert.

Nach der Zerlegung des Berechnungs-Prozesses in mehrere Berechnungs-Pakete 12-i und nach der Spezifikation von Regeln für jedes Berechnungs-Paket 12-i kann eine Datenstruktur erstellt werden, vorzugsweise eine Datenstruktur in tabellarischer Form, die die jeweiligen Berechnungs-Pakete 12-i, die Synchronisations-Punkte und die Regeln, einschließlich der Anzahl der Wiederholungen für diese Regel umfasst. Der Berechnungs-Prozess ist damit auf einen Satz von parametrisierten Regeln abgebildet worden. Die Regeln kennzeichnen die Berechnungs-Pakete 12-i und deren logische Abhängigkeiten bzw. deren Interdependenzen.

Der Berechnungs-Prozess wird erfindungsgemäß in eine Ablauf-Struktur restrukturiert. Diese Ablauf-Struktur basiert auf den einzelnen Berechnungs-Paketen und deren logischen Voraussetzungen bzw. Synchronisations-Punkten.

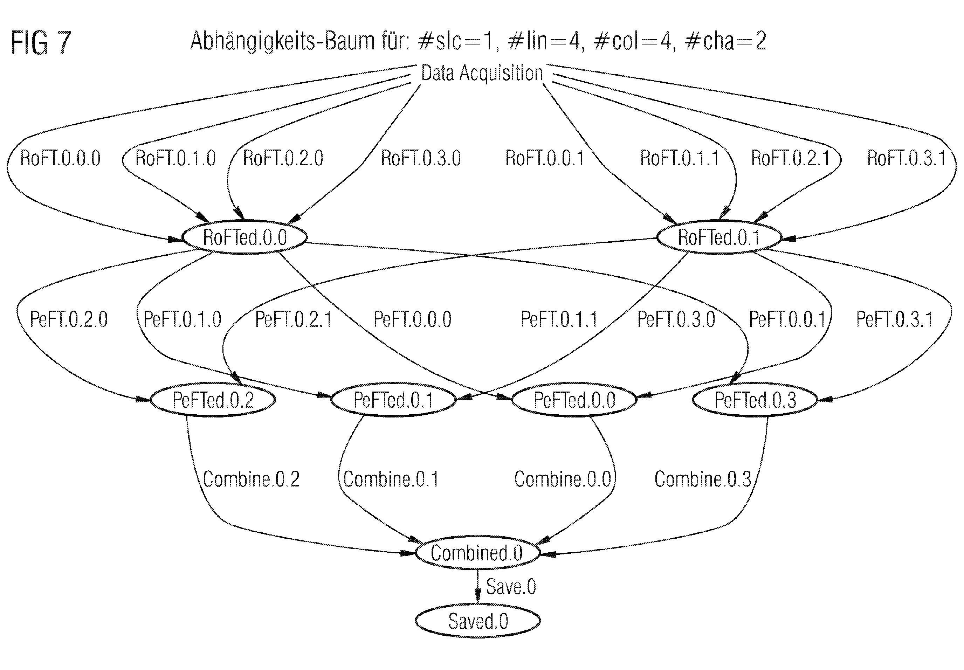

Die Ablauf-Struktur kann in einer baumartigen Datenstruktur dargestellt. Fig.7 zeigt beispielhaft einen solchen "Abhängigkeitsbaum". Die Kanten des Baumes kennzeichnen die Berechnungs-Pakete 12-i. Die Knoten kennzeichnen die jeweiligen Abhängigkeiten bzw. die Synchronisations-Punkte.

Bestimmte Synchronisations-Punkte sind reine Kollektor-Punkte und dienen lediglich zur Erfassung des jeweils ausgeführten Berechnungs-Paketes 12-i. Wenn dieser Synchronisations-Punkt von der Blockade freigeschaltet wird, kennzeichnet dies, dass die Berechnung fertiggestellt ist und die jeweilige Schicht gespeichert wird. Dies ist in Fig.7 mit dem untersten Element "Saved.0" gekennzeichnet.

Des weiteren gibt es bestimmte Synchronisations-Punkte, die reine Blocker sind. Dies betrifft solche Synchronisations-Punkte, die abhängig sind von der Erfassung der Daten und nicht von der erfolgreichen Ausführung anderer Berechnungs-Pakete 12-i. Sie können auf den Zustand "unblockiert" voreingestellt sein, so dass deren Ausführung unmittelbar beginnt. Die Ausführung dieser Berechnungs-Pakete hängt unmittelbar von den erfassten Daten ab und wird von dem Datenerfassungs-Thread initiiert, nachdem die Datenerfassung stattgefunden hat. Diese Knoten sind in der beispielhaften Darstellung in Fig.7 mit dem Begriff "Data-Acquisition" gekennzeichnet.

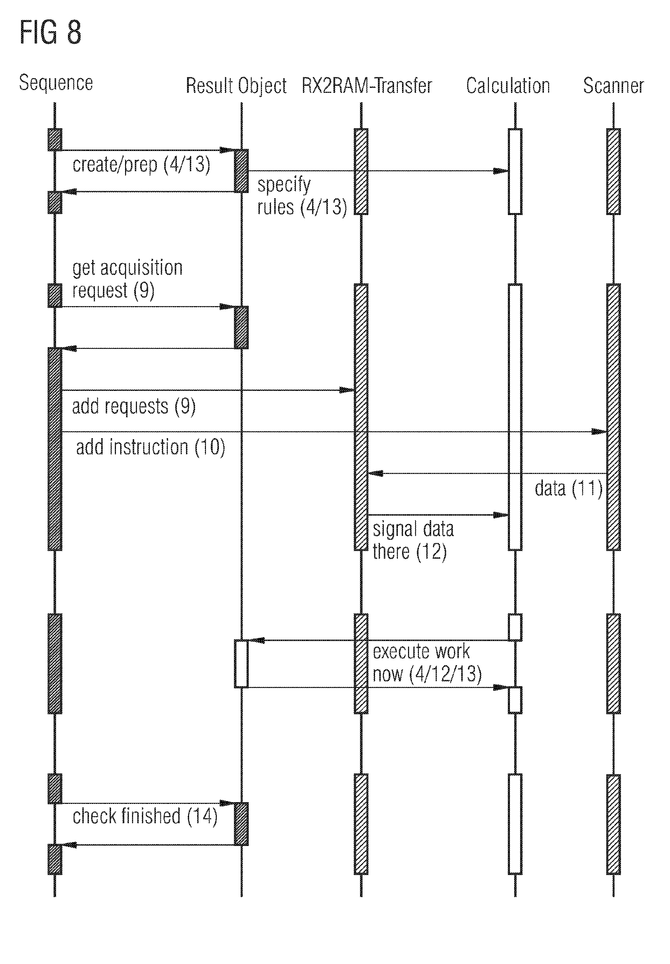

In Fig.8 ist beispielhaft die erfindungsgemäße Synchronisation zwischen einer Sequenz bzw. einer Messdaten-Sequenz und einer Berechnungs- bzw. Calculation-Einheit dargestellt. Innerhalb einer Vorbereitungs- oder einer Ablaufphase kann die Sequenz entscheiden, ob sie ein bestimmtes Ergebnisobjekt (Result-Object) erzeugen möchte. Während der Vorbereitungsphase wird für das Ergebnisobjekt ein Satz von parametrisierten Regeln spezifiziert, um den Rekonstruktions-Algorithmus zu beschreiben. Nachdem die Vorbereitungsphase für das Ergebnisobjekt abgeschlossen worden ist, kann die Sequenz Messdaten MD anfordern.

Da die Sequenz alleine das Einlesen eines bestimmten Scans für ein Ergebnisobjekt triggert, muss sie Information über dieses Ergebnisobjekt haben. Dazu wird eine Akquisitions-Anfrage für das jeweilige Ergebnisobjekt generiert. Eine solche Anfrage kann folgende Daten beinhalten:
- Der Umfang der zu erfassenden Daten, z.B. die Größe des Scans und die Anzahl der Kanäle,
- die Zieladresse der Daten für jeden Kanal,
- ein eineindeutiger Identifikator für die gemessenen Daten.

Die Akquisitions-Anfrage wird verwendet, um eine Anfrage an die Schnittstelle zu dem Scanner des MR-Gerätes zu bilden. Insbesondere für den "RX-to-RAM-Daten-Transfer-Thread", um einen Auslesebefehl für den Scanner zu erzeugen.

Nachdem die Daten von dem Scanner erfasst worden sind und über die Schnittstelle übertragen worden sind, informiert der Transfer-Thread die Berechnungs-Einheit 12 über die Tatsache, dass die Daten erfasst worden sind. Er leitet den eineindeutigen Identifikator für diese Daten, den er von dem Ergebnisobjekt erhalten hat, an die Berechnungs-Einheit 12 weiter. Die Ausführung einer Datenerfassung über den Scanner ist keine notwendige Voraussetzung für ein Ergebnisobjekt. Es können auch beliebige andere Berechnungs-Prozesse, die über die Regeln eingeführt worden sind, ausgeführt werden. Die Berechnungs-Einheit 12 ist verantwortlich für die Organisation und Ausführung der jeweiligen Berechnungs-Pakete 12-i. Falls die Berechnungs-Einheit (bzw. Caculation) 12 entscheidet, eine bestimmte Berechnung auszuführen, so ruft sie eine entsprechende Methode für das Ergebnis-Objekt auf. Die eigentliche Berechnung ist für den jeweiligen Anwender völlig gekapselt. Die Berechnungs-Einheit 12 liest die jeweiligen Regeln.

Durch das erfindungsgemäße Verfahren wird es möglich, auch solche Berechnungs-Prozesse zu berücksichtigen, die auf Messdaten MD basieren, die von unterschiedlichen Quellen stammen. Denn MR-Messungen erfassen typischerweise Eingabedaten für unterschiedliche Ausgabe-Ergebnisse, also für unterschiedliche Berechnungs-Prozesse mit unterschiedlichen Daten und in unterschiedlicher Reihenfolge. Die Organisation bzw. Verwaltung und die Ausführung der Berechnung liegt innerhalb der Verantwortung der Berechnungs-Einheit 12. Die Parallelisierung der beiden Prozesse, die Implementierung der Algorithmen und die Verwaltung des Speichers liegt im Verantwortungsbereich des Ergebnisobjektes. Erfindungsgemäß wird der restrukturierte und parallelisierte Berechnungs-Prozess über parametrisierte Regeln beschrieben.

Ein Vorteil der Verwendung der erfindungsgemäßen Datenstruktur in Form von Abhängigkeitsbäumen erlaubt es, den Vorgang der Parallelisierung mit üblichen Testverfahren zu testen(insbesondere auf Konvergenz auf ein Blatt in dem Baum, also daraufhin zu testen, ob die restrukturierte Ablaufstruktur auch genau ein Ergebnis liefert), was grundsätzlich unabhängig von dem jeweils verwendeten Algorithmus bzw. Berechnungs-Prozess ist. Darüber hinaus ist es möglich, geeignete Maßnahmen zu ergreifen, falls ein Fehler auftritt, der beispielsweise durch eine fehlende oder fehlerhafte Erfassung eines Scans verursacht worden ist. Mit der erfindungsgemäßen Lösung wird es möglich, eine Meldung auszugeben, die angibt, welcher Scan für welchen Datensatz fehlt.

Durch die erfindungsgemäße Restrukturierung des Berechnungs-Prozesses in einer standardisierten Form ist es möglich, dass das Verfahren auf beliebige Bild-Rekonstruktions-Prozesse angewendet werden kann und keinerlei Voraussetzungen im Hinblick auf die Reihenfolge der Datenerfassung machen muss. Dies kann vorteilhafter Weise die Flexibilität und die Stabilität des Berechnungs-Prozesses erhöhen. Aufgrund der Restrukturierung des Berechnungs-Prozesses in die erfindungsgemäße Ablauf-Struktur, die die logischen Abhängigkeiten der jeweiligen Prozesse bzw. deren Interdependenzen umfasst, ist eine externe Synchronisation nicht mehr notwendig.

In einer alternativen Ausführungsform betrifft der Berechnungs-Prozess nicht die MR-Bild-Rekonstruktion, sondern beispielsweise eine Anfrage eines Anwenders für die Nachbearbeitung von Mess-Ergebnissen. Dieses Post-Processing kann nach demselben Verfahren ausgeführt werden, so dass das Post-Processing berechnet wird und abläuft, noch während die Daten von einem lokalen Speicher oder einer Datenbank abgerufen werden.

Eine vorteilhafte Weiterbildung der Erfindung betrifft die Steuerung der Datenerfassung. Aufgrund der erfindungsgemäßen Synchronisation zwischen Erfassungs- und Berechnungs-Prozess ist es möglich, dass der Berechnungs-Prozess bereits spezifiziert, welche Daten von dem Erfassungs-Prozess erfasst werden müssen. Damit kann der Erfassungs-Prozess unmittelbar an die Erfordernisse des Berechnungs-Prozesses angepasst gesteuert werden. Diese Weiterbildung macht vor allem für solche Erfassungsverfahren Sinn, bei welchen die zeitliche Reihenfolge der Messdatenerfassung keinen Einfluss auf die Bildqualität hat, wie z.B. bei einer Computertomographie.

In einer anderen Weiterbildung der Erfindung kann jedes Berechnungs-Paket 12-i mit einer Priorität gekennzeichnet werden, so dass die jeweiligen Berechnungs-Pakete 12-i im Hinblick auf ihre Priorität bevorzugt oder erst zeitlich verzögert oder gar nicht bearbeitet werden.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Für einen einschlägigen Fachmann ist es insbesondere offensichtlich, dass die Erfindung teilweise oder vollständig in Soft- und/oder Hardware und/oder auf mehrere physikalische Produkte - dabei insbesondere auch Computerprogrammprodukte - verteilt realisiert werden kann.

## Patentansprüche

1. Verfahren zur Optimierung eines kombinierten Erfassungs- und Berechnungsprozesses, insbesondere eines Bilddatenerfassungs- und Berechnungsprozesses, wobei der Erfassungsprozess Messdaten (MD) erfasst und wobei der Berechnungsprozess auf den erfassten Messdaten (MD) basiert, während parallel zur Ausführung des Berechnungsprozesses weitere Messdaten (MD) erfasst werden können, wobei die beiden Prozesse über eine Schnittstelle (11) kommunizieren, mit folgenden Verfahrensschritten:
- Zerlegen des Berechnungsprozesses in Berechnungspakete (12-1, 12-2,... .12-n)
- Definieren von Regeln für jedes Berechnungspaket (12-i), indem alle logische Abhängigkeiten zwischen den Berechnungspaketen (12-1, 12-2,...12-n) festgelegt werden, insbesondere, welche Voraussetzungen für die Ausführung des jeweiligen Berechnungspaketes (12-i) erfüllt und/oder welche und wie viele Messdaten (MD) erfasst sein müssen
- Restrukturieren des Berechnungsprozesses in eine Ablauf-Struktur basierend auf den Berechnungspaketen (12-i) und auf deren Regeln
- Steuern des Berechnungsprozesses anhand der Ablauf-Struktur, indem automatisch zum frühestmöglichen Zeitpunkt das jeweilige Berechnungspaket (12-i) ausgeführt wird.

2. Verfahren nach Anspruch 1,
**dadurch gekennzeichnet, dass**
zumindest zwei Synchronisationspunkte, insbesondere genau zwei Synchronisationspunkte, für jeweils ein Berechnungspaket (12-i) definiert werden:
- ein Blocker, der alle Voraussetzungen für die Ausführung des Berechnungspaketes (12-i), alle logischen Abhängigkeiten des Berechnungspaketes (12-i) von anderen Instanzen, insbesondere von dem Erfassungsprozess, berücksichtigt, und
- ein Kollektor, der die Ausführung des jeweiligen Berechnungspaketes (12-i) erfasst, so dass
das jeweilige Berechnungspaket (12-i) automatisch zum frühestmöglichen Zeitpunkt ausgeführt wird, sobald der Blocker für das Berechnungspaket (12-i) nicht mehr blockiert ist.

3. Verfahren nach zumindest einem der vorstehenden Ansprüche,
**dadurch gekennzeichnet, dass**
der Berechnungsprozess unabhängig von einer Reihenfolge und unabhängig von einer Geschwindigkeit der Messdatenerfassung gesteuert wird.

4. Verfahren nach zumindest einem der vorstehenden Ansprüche,
**dadurch gekennzeichnet, dass**
der Berechnungsprozess auf einem beliebigen Berechnungsalgorithmus basieren kann.

5. Verfahren nach zumindest einem der vorstehenden Ansprüche,
**dadurch gekennzeichnet, dass**
der Berechnungsprozess ein Rekonstruktionsprozess ist und Bilddaten generiert, insbesondere Magnet-Resonanz-Bilddaten, Ultraschall-Bilddaten und/oder computer-tomographische Daten.

6. Verfahren nach zumindest einem der vorstehenden Ansprüche,
**dadurch gekennzeichnet, dass**
die Regeln eine vollständige Beschreibung eines dem Berechnungsprozess zugrundeliegenden Problems sind.

7. Verfahren nach zumindest einem der vorstehenden Ansprüche,
**dadurch gekennzeichnet, dass**
die Berechnungspakete und/oder die Prozesse bei der Messdatenerfassung auf mehrere parallel laufenden Threads aufgeteilt werden.

8. Verfahren nach zumindest einem der vorstehenden Ansprüche,
**dadurch gekennzeichnet, dass**
das Verfahren sich selbst organisiert und keine externe Synchronisation zwischen dem Erfassungs- und dem Berechnungsprozess erfordert.

9. Verfahren nach zumindest einem der vorstehenden Ansprüche,
**dadurch gekennzeichnet, dass**
das Verfahren zusätzlich den Erfassungsprozess steuert, indem die Ablauf-Struktur des Berechnungsprozesses anhand der Regeln ausgewertet werden, so dass die Berechnungspakete (12-i) bei Bedarf ihre notwendigen Messdaten (MD) anfordern und einen entsprechenden Messvorgang triggern.

10. Verfahren nach zumindest einem der vorstehenden Ansprüche,
**dadurch gekennzeichnet, dass**
die Rechnerarchitektur für das Verfahren ein Single - oder eine Multiprozessorsystem ist und/oder dass das Verfahren optimal an die jeweilige Rechnerarchitektur angepasst werden kann.

11. Verfahren nach zumindest einem der vorstehenden Ansprüche,
**dadurch gekennzeichnet, dass**
zumindest einer der Verfahrensschritte - und vorzugsweise alle Verfahrenschritte - automatisch erfolgt/en.

12. Verfahren nach zumindest einem der vorstehenden Ansprüche,
**dadurch gekennzeichnet, dass**
das Ergebnis des Verfahrens, gegebenenfalls Zwischenergebnisse und/oder die generierte Ablauf-Struktur - z.B. in Form eines gerichteten Graphen, insbesondere eines Abhängigkeitsbaumes, oder einer Tabelle - angezeigt werden.

13. Verfahren nach zumindest einem der vorstehenden Ansprüche,
**dadurch gekennzeichnet, dass**
das Verfahren Zwischenergebnisse und/oder Ergebnisse des Erfassungsprozesses und/oder des Rekonstruktionsprozesses in einen Pufferspeicher speichert und/oder zur Echtzeitsteuerung des Erfassungsprozesses verwendet.

14. Datenstruktur für einen Berechnungsprozess, insbesondere für einen Rekonstruktionsprozess für ein MR-Bild, zur Verwendung bei einem Verfahren nach zumindest einem der Ansprüche 1 bis 13 mit:
- einer Vielzahl von Berechnungspaketen (12-i), die eine in Subaufgaben zerlegte Aufgabe des Berechnungsprozesses ausführen
- zumindest zwei Synchronisationspunkten, insbesondere genau zwei Synchronisationspunkten, für jeweils jedes Berechnungspaket (12-i), in denen alle logischen Abhängigkeiten zwischen den Berechnungspaketen (12-i) definiert werden und in denen die Ausführung des jeweiligen Berechnungspaketes (12-i) registriert wird
- Zuordnung zwischen jeweils einem Berechnungspaket (12-i) und den beiden Synchronisationspunkten.

15. Vorrichtung zur Optimierung einer kombinierten Erfassungs- und Berechnungseinheit (10, 12), insbesondere einer Bilddatenerfassungs- und Berechnungseinheit, wobei die Erfassungseinheit (10) Messdaten (MD) erfasst und wobei die Berechnungseinheit (12) auf den erfassten Messdaten (MD) basiert, während parallel zur Ausführung der Berechnungseinheit (12) weitere Messdaten (MD) von der Erfassungseinheit (12) erfasst werden können, wobei eine Schnittstelle (11) vorgesehen ist, über die die beiden Einheiten (10, 12) kommunizieren, umfassend:
- eine Vielzahl von Berechnungsmodulen, die dazu bestimmt sind, die in Berechungspakete (12-i) zerlegte Aufgabe der Berechnungseinheit (12) auszuführen und die auf verschiedene Threads aufgeteilt werden können
- zumindest eine Synchronisationseinheit (14), die dazu bestimmt ist, für jedes Berechnungspaket zu bestimmen, welche logischen Abhängigkeiten von anderen Berechnungspakten (12-i) und für andere Berechnungspakten (12-i) bestehen
- eine Datenstruktur für den Ablauf der Berechnungspakete (12-i) der Berechnungseinheit (12), basierend auf der Synchronisationseinheit (14)
- zumindest eine Steuerungseinheit (16), die dazu bestimmt ist, die Berechnungseinheit (12) anhand der Datenstruktur zu steuern, indem automatisch zum frühestmöglichen Zeitpunkt das jeweilige Berechnungspaket (12-i) ausgeführt wird, sobald dessen Synchronisationseinheit (14) dies veranlasst.

16. Vorrichtung zur Ausführung des Verfahrens mit den Merkmalen nach zumindest einem der Ansprüche 1 bis 13.
